# EUROPEAN PATENT SPECIFICATION

(11) **EP 0 773 783 B1**
(45) Date of publication and mention of the grant of the patent: **28.11.2001**
(21) Application number: 95927705.4
(22) Date of filing: 21.07.1995
(51) Int. Cl.: A61K 31/56, A61K 9/20

(54) **PREPARATION OF FUSIDIC ACID TABLETS**
VERFAHREN ZUM HERSTELLEN VON FUSIDINSÄURETABLETTEN
PREPARATION DE COMPRIMES A BASE D'ACIDE FUSIDIQUE

(30) Priority: 26.07.1994 GB 9415048
(43) Date of publication of application: 21.05.1997
(73) Proprietor: LEO PHARMACEUTICAL PRODUCTS LTD. A/S (LOVENS KEMISKE FABRIK PRODUKTIONSAKTIESELSKAB), 2750 Ballerup (DK)
(72) Inventor: RASMUSSEN, Erik, Preben, DK-4000 Roskilde (DK)
(74) Representative: Golding, Louise Ann
(86) International application number: EP9502904
(87) International publication number: WO9603128

(56) References cited:
- EP-A- 0 300 073
- DE-A- 2 443 431
- CHEMICAL ABSTRACTS, vol. 80, no. 6, 11 February 1974, Columbus, Ohio, US; abstract no. 30673, & KHIM.-FARM. ZH., vol.7, no.8, 1973 pages 50 - 53 L.K.GRAKOVSKAYA ET AL. 'GRANULATION OF FUSIDATE SODIUM'
- CHEMICAL ABSTRACTS, vol. 78, no. 6, 12 February 1973, Columbus, Ohio, US; abstract no. 33893, & KHIM.-FARM. ZH., vol.6, no.10, 1972 pages 52 - 56 M.L.EZERSKIT ET AL. 'USE OF JET GRINDING FOR PRODUCING PHARMACEUTICAL POWDERS'

## Description

Fusidic acid is an antibiotic which is used both enterally and parenterally.

When used as an oral solid form, it is used in the form of its sodium salt which is readily soluble in e.g. water and ethanol.

At the beginning of the eighties, fusidic acid sodium salt was used in capsules as well as in tablets. The tablets were enteric coated, and were the preferred form due to certain gastric side effects of the capsules.

When the tablets (diameter larger than 6-7 mm) were administered close to meals, large individual variations in the blood concentrations were observed, probably due to the tablets 'following the food', dependent on the time of passage of the food through the stomach, this factor being different from individual to individual. The active fusidic acid sodium salt was not released before the tablets reached the part of the gastrointestinal tract in which the enteric coating would be dissolved, and depending on the time of passage through the stomach together with the food and the pH in the gastrointestinal tract, this led to unpredictable variations in the blood concentration of the patient undergoing treatment.

To avoid these adverse differences in the blood concentration, it seemed necessary to try to produce tablets without the enteric coating. However, in order to avoid or lessen gastric side effects in the upper part of the gastrointestinal tract, a quickly disintegrating tablet was needed, and said tablet should be film coated to avoid the unpleasant taste of fusidic acid sodium salt, the film coating using organic solvents.

Fusidic acid sodium salt exhibits a large powder volume and poor flow characteristics, together with an abrasive effect on the die walls, thus making compacting of the tablet contents in tablet presses ('slugging') impossible or at least very difficult, the only possibility left being a granulation before tabletting.

The problem was partly solved by using a wet granulation: comminution, blending, remilling and lubrication, using organic solvents in the process (e.g. chlorinated hydrocarbons).

Due to environmental and/or toxicological considerations, it became desirable to avoid the use of these types of organic solvents.

Wet granulation by moistening the fusidic acid sodium salt with water or alcohol was not possible due to the high solubility of the salt in these solvents. It was instead tried to spray the said solvents onto the salt, but this gave also a very porous and voluminous material which was extremely difficult to dose into the dies of the tabletting machine, and worse, it led to tablets with poor stability.

Acetone would be a better solution, but due to the high inflammability of this solvent, the process has to be performed in separate and secured processing plants.

It has now turned out that the necessary granulation before tabletting can be accomplished by using a dry granulation process using a special processing equipment known as a roller compactor or 'chilsonator'. These machines compress premixed powders between two counterrotating rollers under extreme pressure. The resulting material is in the form of a brittle ribbon, sheet or piece depending on the configuration of the roller which is far denser than the wet granulates referred to above which have a bulk density of about 0.40 g/cm³. The compressed material is reduced to the proper size for tablet granulation purposes to give a granulate having a bulk density in the range 0.45 to 0.9 (preferably 0.5-0.7) g/cm³ and the final tablets are produced from this material.

According to the present invention therefore we provide a process for the preparation of tablets of fusidic acid sodium salt without an enteric coating, in which dry powdered fusidic acid sodium salt is compressed in a roller compactor and the compacted material so produced is size-reduced to form a granulate having a bulk density in the range 0.45 to 0.9 g/cm³ which is then formed into tablets.

In a further aspect, the invention also provides tablets of fusidic acid sodium salt without an enteric coating, formed from a dry granulate of compacted powdered fusidic acid sodium salt, which granulate has a bulk density of 0.45 to 0.9 g/cm³. In another aspect, the invention provides a granulate for the preparation of tablets as just defined, which granulate comprises compacted powdered fusidic acid sodium salt and has a bulk density of 0.45 to 0.9 g/cm³.

The invention also provides a process for the preparation of tablets of fusidic acid sodium salt without an enteric coating, in which compacted powdered fusidic acid sodium salt having a bulk density of 0.45 to 0.9 g/cm³ is formed into tablets.

The mean particle size of the granulate formed from the compacted material is preferably in the range 100 *µ*m to 300 *µ*m. The bulk density of the granulate is preferably 0.5 to 0.7 g/cm³.

The use of a α-tocopherol coated active material is also possible and leads to even better storage stability of the tablets.

Tests carried out with these new tablets show a bioavailability of the same magnitude as with the conventional tablets, and thus the new tablets combine the previous advantageous effect in human therapy with a more environmentally friendly, and safer, production method.

In addition hereto, it has turned out that the new tablets, with and without α-tocopherol coated active material, exhibit a better storage stability than the tablets produced according to the traditional (or previous) wet granulation method.

The following examples are given by way of illustration only:

### Example 1

Powdered fusidic acid sodium salt was dry granulated. The compacted material had a density of 0.46 g/cm³. The particle size of the granulate was reduced to an average of about 0.160 mm by crushing and sieving. The granulate was compressed into tablets containing 250 mg fusidic acid sodium salt per tablet (Batch No. 9106651). The tablets were film coated with hydroxypropylmethylcellulose.

### Example 2

Example 1 was repeated using α-tocopherol coated fusidic acid sodium salt as starting material (Batch No. 9106151). The compacted material had a bulk density of 0.46 g/cm³ and the average particle size of the granulate after size reduction was 0.250 mm.

### Example 3

The tablets of Examples 1 and 2 and tablets formed from a wet granulated powder (prepared by using a high speed granulator) having a bulk density of about 0.40 g/cm³ (Batch No. 14360 R) were stored at the indicated temperatures in glass bottles provided with polypropylene caps. The analytical results obtained by the reversed phase HPLC method described in Ph.Eur., 2nd Ed., p. II, 798 are shown in the following table:

### Fusidic acid tablets - storage comparison

| Storage | | Degradation Products, in % | | |
|---|---|---|---|---|
| Temp. °C | Time, months | Batch 14360R | Batch 9106651 | Batch 9106151 |
| 20 | 0 | 0.3 | 0.9 | 0.5 |
| 20 | 6 | 0.7 | 1.2 | 0.9 |
| 20 | 12 | 1.0 | 2.0 | 0.5 |
| 20 | 24 | 2.5 | 0.9 | 0.5 |
| 20 | 36 | 2.8 | 2.1 | 0.4 |
| 20 | 48 | 5.2 | n.d. | 0.6 |
| 30 | 0 | 0.3 | 0.9 | 0.5 |
| 30 | 6 | 0.9 | | 0.9 |
| 30 | 12 | 3.4 | 0.7 | 0.5 |
| 30 | 24 | 11.8 | 1.6 | 0.7 |
| 30 | 36 | 14.2 | 2.6 | 0.7 |
| 40 | 0 | 0.3 | 0.9 | 0.5 |
| 40 | 3 | 0.5 | 2.1 | 0.5 |
| 40 | 6 | 1.7 | 1.6 | 0.9 |
| 40 | 12 | 9.0 | 1.9 | 2.0 |

A pronounced improvement is obtained at all the tested storage temperatures, but is, of course, most apparent at the temperatures of 30°C and 40°C.

## Claims

1. A process for the preparation of tablets of fusidic acid sodium salt without an enteric coating, in which dry powdered fusidic acid sodium salt is compressed in a roller compactor and the compacted material so produced is size-reduced to form a granulate having a bulk density of 0.45 to 0.9 g/cm³ which is then formed into tablets.

2. A process according to claim 1 in which the particle size of said granulate is 100 to 300 *µ* m.

3. A process according to claim 1 or claim 2 in which the bulk density of the granulate is 0.5 to 0.7 g/cm³.

4. A process according to any preceding claim in which the powdered fusidic acid sodium salt is coated with α-tocopherol.

5. A process according to any preceding claim in which the tablets produced are film coated.

6. Tablets of fusidic acid sodium salt without an enteric coating, formed from a dry granulate of compacted powdered fusidic acid sodium salt, which granulate has a bulk density of 0.45 to 0.9 g/cm³.

7. A granulate for the preparation of tablets according to claim 6, which granulate comprises compacted powdered fusidic acid sodium salt and has a bulk density of 0.45 to 0.9 g/cm³.

8. A process for the preparation of tablets of fusidic acid sodium salt without an enteric coating, in which compacted powdered fusidic acid sodium salt having a bulk density of 0.45 to 0.9 g/cm³ is formed into tablets.

9. Tablets according to claim 6 or a granulate according to claim 7 or a process according to claim 8, in which the said bulk density is 0.5 to 0.7 g/cm³.

## Patentansprüche

1. Verfahren für die Herstellung von Fusidinsäure-Natriumsalz-Tabletten ohne eine magensaftresistende Beschichtung, bei dem trockenes pulverförmiges Fusidinsäure-Natriumsalz in einem Walzverdichtungsgerät komprimiert wird und das so hergestellte komprimierte Material in der Größe reduziert wird, um ein Granulat zu bilden, das eine Schüttdichte von 0,45 bis 0,9 g/cm³ aufweist und das dann zu Tabletten geformt wird.

2. Verfahren nach Anspruch 1, bei dem die Partikelgröße des Granulats 100 bis 300 *µ*m beträgt.

3. Verfahren gemäß Anspruch 1 oder Anspruch 2, bei dem die Schüttdichte des Granulats 0,5 bis 0,7 g/cm³ beträgt.

4. Verfahren nach einem der vorstehenden Ansprüche, bei dem das pulverförmige Fusidinsäure-Natriumsalz mit α-Tocopherol beschichtet ist.

5. Verfahren nach einem der vorstehenden Ansprüche, bei dem die hergestellten Tabletten filmbeschichtet sind.

6. Tabletten aus Fusidinsäure-Natriumsalz ohne eine magensaftresistente Beschichtung, die aus einem trockenen Granulat aus komprimiertem pulverförmigen Fusidinsäure-Natriumsalz gebildet sind, wobei das Granulat eine Schüttdichte von 0,45 bis 0,9 g/cm³ aufweist.

7. Granulat für die Herstellung von Tabletten nach Anspruch 6, wobei das Granulat komprimiertes pulverförmiges Fusidinsäure-Natriumsalz enthält und eine Schüttdichte von 0,45 bis 0,9 g/cm³ aufweist.

8. Verfahren für die Herstellung von Tabletten aus Fusidinsäure-Natriumsalz ohne eine magensaftresistente Beschichtung, bei dem komprimiertes pulverförmiges Fusidinsäure-Natriumsalz mit einer Schüttdichte von 0,45 bis 0,9 g/cm³ zu Tabletten geformt wird.

9. Tabletten nach Anspruch 6 oder Granulat nach Anspruch 7 oder Verfahren nach Anspruch 8, bei dem die Schüttdichte 0,5 bis 0,7 g/cm³ beträgt.

## Revendications

1. Procédé pour la préparation de comprimés à base de sel de sodium d'acide fusidique sans revêtement kératinisé, dans lequel le sel de sodium d'acide fusidique en poudre sec est comprimé dans un compacteur à rouleau et le matériau compacté ainsi produit est fragmenté pour former un granulé ayant une masse volumique en vrac de 0,45 à 0,9 g/cm³ qui est ensuite moulé en comprimés.

2. Procédé selon la revendication 1, dans lequel la taille des particules dudit granulé est de 100 à 300 µm.

3. Procédé selon la revendication 1 ou 2, dans lequel la masse volumique en vrac du granulé est de 0,5 à 0,7 g/cm³.

4. Procédé selon l'une quelconque des revendications précédentes, dans lequel le sel de sodium d'acide fusidique en poudre est revêtu d'α-tocophérol.

5. Procédé selon l'une quelconque des revendications précédentes, dans lequel les comprimés produits sont revêtus d'une pellicule.

6. Comprimés de sel de sodium d'acide fusidique sans revêtement kératinisé, formés à partir d'un granulé sec de sel de sodium d'acide fusidique en poudre compacté, ledit granulé ayant une masse volumique en vrac de 0,45 à 0,9 g/ cm³.

7. Granulé pour la préparation de comprimés selon la revendication 6, ledit granulé comprenant un sel de sodium d'acide fusidique en poudre compacté et a une masse volumique en vrac de 0,45 à 0,9 g/cm³.

8. Procédé pour la préparation de comprimés à base de sel de sodium d'acide fusidique sans revêtement kératinisé, dans lequel le sel de sodium d'acide fusidique en poudre compacté ayant une masse volumique en vrac de 0,45 à 0,9 g/ cm³ est moulé en comprimés.

9. Comprimés selon la revendication 6 ou granulé selon la revendication 7, ou procédé selon la revendication 8, dans lesquels ladite masse volumique en vrac est de 0,5 à 0,7 g/ cm³.
